# EUROPEAN PATENT APPLICATION

(11) **EP 4 759 394 A1**
(43) Date of publication of application: **17.06.2026**
(21) Application number: 26158864.4
(22) Date of filing: 27.12.2023
(51) Int. Cl.: B01D 15/38, B01D 15/36, B01D 15/18

(54) **METHOD FOR SELECTIVE SEPARATION OF AMPHOTERIC SPECIES**

(30) Priority: 30.12.2022 DE 102022135036; 18.04.2023 DE 102023109774; 13.05.2023 WO PCT/EP2023/062862
(62) Divisional of application: 23840983.3
(71) Applicant: i3 Membrane GmbH, 22761 Hamburg (DE)
(72) Inventor: RENFREW, Kenneth, North Carolina, 28078 (US); BRINKE-SEIFERTH, Stephan, 22085 Hamburg (DE); SCHMITT, Florian, 26131 Oldenburg (DE); KOCH, Ina, 01900 Großröhrsdorf (DE); BERGJOHANN, Ulrike, 55234 Nack (DE); SKORUPA, Kilian, 01127 Dresden (DE)
(74) Representative: Raffay & Fleck

(57) **Abstract**

The invention relates to a method for selective separation of amphoteric species, in particular biomolecules or biomolecule complexes, e.g., vectors containing molecule, based on their isoelectric points. For selective separation, a charged porous surface is provided being electrically conductive and/or comprising an electrically conductive coating or layer. Fluid containing the amphoteric species is brought in contact with the surface, thereby absorbing species to the surface due to an attractive interaction force. Thereafter, at least parts of the adsorbed species are released by applying a release voltage chosen so that the net interaction force between the surface and adsorbed species is switched from attractive to repulsive.

Selectivity is achieved by selective adsorption, thus at least one species being not absorbed, and/or selective desorption, thus selectively releasing some species previously adsorbed with others remaining bound to the surface, preferably selectively releasing solely one species of the previously adsorbed.

## Description

The Invention relates to a method for selective separation of amphoteric species, in particular (bio-)molecules, e.g., proteins, amino acids or nucleic acids, or molecule complexes, e.g. vectors, in particular capsids, plasmids or vesicles, with respect to their isoelectric point, respectively molecule(s), e.g., genomes, contained within the molecule complexes, in particular vectors, by means of selective (electro-)adsorption and electro-desorption, especially in order to prepare high-purity samples of desired amphoteric species, in particular vectors and/or biomolecules.

Generally, biomolecules are sought-after species for medical applications, e.g., proteins to be used as drugs or alike. As such, the desired biomolecules are to be produced with high purity, thus need to be separated from impurities resulting from the formation of those biomolecules. Such separation is often performed by bind-elute processes, in which the desired molecule is bound to, e.g., an exchange resin, gel or membrane and subsequently eluted from said resin, gel or membrane by means of pH value change, e.g., acidic elution using acetate buffer solutions at pH 3, and/or by using salt solutions to displace the desired biomolecules with salt ions. Both of those elution methods, may lead to harsh conditions which may be harmful for any biomolecule, e.g., may lead to agglomeration or denaturation of the desired species, especially with respect to proteins. Examples for such methods are isoelectric focusing or ion-exchange-chromatography.

Particularly interesting species in the field of biomedicine are vectors. Such vectors are commonly used as tools for delivering molecules, in particular biologically active substances, into cells. As such, vectors play a key role in gene therapy and in the field of vaccines or other therapeutics, particularly including, e.g., personalized cancer therapeutics. For those purposes, vectors, are prepared containing molecule(s), in particular in the form of biologically active substances, e.g., containing genomes in the form of deoxyribonucleic acid (DNA), single-strain deoxyribonucleic acid (ssDNA), ribonucleic acid (RNA) or single-strain ribonucleic acid (ssRNA).

As viral vectors, e.g., vectors based on retroviruses, lentiviruses, adenoviruses or adeno-associated viruses are commonly used. For all of those, a capsid protein is forming a shell in which a molecule, especially a biologically active substance like a medicine and/or a genome in the form of DNA, RNA, ssDNA or ssRNA can be placed. Consequently, the capsid is, e.g., used as a means for delivering biologically active substances, and in particular as a protective shell for the molecule to be delivered, in particular may be used as vehicle to artificially carry the biologically active substance, e.g., a nucleic sequence, usually DNA or RNA, into a cell, e.g., for expression or replication. Plasmids, often small circular, mostly double-stranded DNA or RNA molecules which are mostly found in bacteria are commonly used as vectors as well. With respect to gene therapy, plasmids are mostly used by integrating a desired genome sequence into the plasmid itself. The plasmid may then migrate into a cell and might participate in replication of the integrated genome sequence or alike.

For gene therapy and other medical applications, it is desired to use high-purity vectors. However, while preparing the vectors containing molecule(s), commonly the vectors may differ in molecule(s) contained, especially regarding amount of molecule and/or the molecule(s) contained. E.g., there might be contained a specific molecule or only a part of such molecule or some vectors may not contain any molecule at all. E.g., when preparing a capsid containing a genome, normally, the vectors gained contain the full genome or a partial genome or even no genome at all. For using such in therapy, it is desired to selectively separate vectors containing the correct, especially full, genome or molecule in a correct amount from vectors which do not fulfill, in particular fully fulfill, those conditions.

For separating vectors containing a full genome from those containing a partial genome or no genome several techniques are known, those being, e.g., Anion Exchange-High Performance Liquid Chromatography (AEX HPLC), capillary isoelectric focusing (cIF), ultracentrifugation (UCF) or gel isoelectric focusing (glF). Of those, AEX HPLC, cIF and glF are used as preparative techniques in the manufacturing of vectors for gene therapy and the like whilst UCF is only common in analytics. In particular, those techniques are not limited to vectors but can be applicable for amphoteric species in general, especially biomolecules or biomolecule complexes.

Both, AEX HPLC and IF methods are based on the overall electrical charge of capsids. This overall electrical charge of the capsids is a function of the amount of genome they contain with respect to the pH value of the surrounding environment. However, the differences in isoelectric points for full and partial and empty capsids are low, corresponding to differences, e.g., in the 0.1 regime.

Within AEX HPLC methods, capsids with cationic overall charge, thus a net overall charge with positive polarity, are adsorbed to chemically negative charged surface, e.g., a resin, containing anionic binding sites. In order to eluate the vectors bound at the anionic binding sites, it is common to either change the pH value of the environment to change the net overall charge of the vectors from being negative to neutral or positive or to use salts to replace the vectors bound by removing them from the binding sites and to effectively block those bindings due to higher affinity, thus releasing the vectors. E.g., MgCl₂ and/or NaCl solutions can be used as eluents. However, the vectors released with such salt solutions must be separated from the salts afterwards since higher concentration of such salts may be harmful in medical applications. Furthermore, elution via change of the pH value of the environment may harm the vector itself, since flocculation of the capsid proteins may occur, especially if the capsid proteins become neutral in charge.

Analogously, isoelectric focusing techniques like gel isoelectric focusing or capillary isoelectric focusing suffer from the same problems and limitations compared to AEX HPLC methods. Within said isoelectric focusing techniques, e.g., a gel consisting of several areas with different pH values is prepared. A solution containing amphoteric biomolecules with different isoelectric points is brought to contact with said gel. The different pH value areas are sorted increasing with a gradient along a single axis. Within said axis an electric field is applied. Then, the amphoteric biomolecules travel with respect to their charge along the electric field. When a molecule reaches an area with a pH value equal to its isoelectric point the amphoteric biomolecules become neutral in charge and stops movement along the axis, thereby separating the biomolecules with respect to their isoelectric point. However, since the biomolecules become neutral in charge, flocculation is likely to occur which may harm the biomolecule.

Moreover, due to the small differences in the isoelectric point aforementioned techniques are often limited with respect to purity. Normally, both AEX HPLC and IF methods can yield preparations with, e.g., capsids containing a full genome being 70% with the rest being mostly capsids with a partial genome or empty capsids. Consequently, it is desirable to achieve higher grades of separation between those vectors to gain capsid preparation with higher purity.

Other methods for separating biomolecules in general comprise filtration via electro-sorption methods. Within those, EP 3 115 099 B1 describes a method for enhancing retention of biomolecules with a metal coated polymer membrane by applying a voltage to the membrane. Moreover, WO 2021/084080 A1 describes a method for electro-desorption of molecules adsorbed to a charged membrane with a metal coating by applying a voltage with opposite polarity to the charge of the membrane to the coating. However, such methods do not provide the selectivity needed with regard to biomolecules which only slightly differ, especially for separating vectors containing either full, partial or no genome.

Based on this, the task is to provide a suitable method for separating amphoteric species, especially biomolecules or biomolecule complexes, in particular vectors, which may differ ever-so-slightly and nevertheless provides high-purity separation, in which desorption via change of the pH value and/or via application of salt solutions is avoided, thus eliminating potential harm of degradation of the separated biomolecules and biomolecule complexes, which especially, if degraded by any means, could provide health risks in medical applications or alike.

This inventive task is solved by a method according to claim 1. The subsequent claims 2 to 25 provide advantageous embodiments of the method according to the invention.

The invention provides a method for selective separation of amphoteric species, in particular with respect to their isoelectric point. The species to be separated consists of at least two specific species. Of those, each specific species possesses a specific isoelectric point. Thereby each specific species has a specific net overall charge at a given pH value, in particular in accordance to their specific isoelectric points. The given pH value may particularly be a predetermined pH value.

The given pH value is preferably in the range of pH°6 to pH°9, preferably in the range of pH°6.5 to 8.5, even preferred in the range of pH°7 to pH°8, which is in general least harmful to species that might be therapeutically used or happen to naturally exist within the human body.

In this context, the term "species" may refer to any (bio-)molecule(s) or (bio- )molecule complexes. (Bio-)molecules which may be separated using the invention may include but are not limited to amino acids, proteins, nucleic acids like RNA or DNA.

(Bio-)Molecule complexes may include but are not limited to vectors containing molecule(s), in particular containing biomolecule(s).

The term "vectors containing molecule" refers to vectors of which at least some of said vectors contain molecule(s), said molecule(s) being different, e.g., regarding amount and/or type, for parts of the vectors, thereby, providing at least two groups of vectors with different contained molecule(s) as the at least two specific species to be separated.

In this context, the term "with different contained molecule" includes different molecules and/or different amounts of molecule(s), in particular different amounts of the same molecule(s) and/or different parts of and/or a different specific molecule. Especially, the term "different amounts of molecule" may include an amount of zero molecule. Consequently, some vectors may not contain any molecule at all, thus "empty" vectors may form a group of vectors or may be part of a group of vectors.

In Particular, the vectors consist of vectors of solely the same type of vectors and/or possess an equal base structure, especially without molecule contained. In particular, the vectors consist of capsids and/or plasmids. Especially, the vectors are viral vectors, e.g., based on retroviruses, lentiviruses, adenoviruses or adeno-associated virus-based vectors.

Any vector possesses a base isoelectric point and thus a base net overall charge at a given pH value, in particular of the surrounding environment. Especially, the base isoelectric point corresponds to the isoelectric point of an empty vector, thus a vector containing no molecule(s). Analogously the base net overall charge is the net overall charge of empty vectors. Said base isoelectric point and the base net overall charge of any specific vector of the vectors containing molecule is shifted by molecule contained in the specific vector. Consequently, a shifted isoelectric point different from the base isoelectric point and a shifted net overall charge at the given pH value different to the base overall charge at the given pH value is yielded due to the molecule contained within the specific vector. Thereby, the at least two groups of vectors are defined, in particular based on the molecule contained in the vectors of each group, especially with regard to different molecules contained and/or different amount of molecule(s) contained. In particular, the different molecules can be molecules of a different type and/or different parts of the same molecule and/or molecules of the same type.

In particular, of the at least two groups of vectors, the first group of vectors possess a first isoelectric point and/or range of first isoelectric points and/or a first overall charge and/or range of first net overall charges at the given pH value and that a second group of vectors possess a second isoelectric point and/or range of second isoelectric points and/or second net overall charge and/or range of second net overall charges at the given pH value. In particular, groups being defined by ranges of isoelectric points and/or ranges of net overall charges, may be inhomogeneous, especially may consist of more than one type of vectors and/or vectors containing different molecules and/or different amounts of molecule(s). Especially, due to molecule contained, an electronic structure of the vectors is influenced. Said influence may lead to the shift in isoelectric point and/or net overall charge of the vectors containing molecule. Especially, the shifted isoelectric points and/or shifted net overall charges might be grouped in ranges, wherein separation of the vectors into groups of vectors falling within those ranges is desired. Especially with respect to undesired impurities separation of those into several groups may be unnecessary. Consequently, if applicable, a range of isoelectric points and/or net overall charges may be defined, which consists of multiple different impurities, e.g., empty and partially filled vectors.

In particular, said at least two groups of vectors are to be separated selectively from each other by performing the method according to the invention.

In yet another embodiment, the method according to the invention may be used for separation of groups of species. Of those groups of species, each group may consist of more than one specific species. In particular such species may be species of equal type with solely minor difference, e.g., biomolecules of equal medical applicability or alike, e.g., enantiomers having no enantiomer specific applications but in particular variants of proteins or alike being part of one protein family, in particular, if those do not need to be separated with respect to use in medical applications. With respect to such groups, the specific isoelectric point and/or specific net overall charge of species of such groups may than be, especially disjunct, ranges of isoelectric points and/or ranges of net overall charges, e.g., one group with a first range and a second group with a different, especially disjunct, range. In particular, such groups of specific species may be defined by such ranges of isoelectric points and/or net overall charges.

The method comprises the steps of
a) providing a charged, in particular chemically and/or electrically charged, porous surface, wherein the charged porous surface is electrically conductive and/or comprises an electrically conductive coating or layer, wherein in particular the conductive layer may - preferably - be integral to and/or integrally formed within the porous surface alternatively it may be a distinct, particularly non-integral, layer;
b) bringing a fluid with the given pH value in contact with the charged porous surface, the fluid containing species, in particular vectors containing molecule, thereby adsorbing at least a first species of the at least two species or at least a first group of species, in particular first vectors or a first group of vectors of the at least two groups of vectors, to the charged porous surface due to an interaction force between the net overall charge of the adsorbed species, in particular vectors, and the charged porous surface, the interaction force being attractive;
c) preferably passing at least parts of the fluid over and/or through the charged porous surface; and
d) applying a release voltage to the electrically conductive charged porous surface and/or the electrically-conductive coating or layer, the release voltage being chosen, so that the net interaction force between the charged surface and at least parts of the adsorbed species, in particular vectors, is switched from attractive to repulsive, thereby releasing said parts of the adsorbed species, in particular vectors.

Advantageously, the porous surface may be formed by one or more filter membrane(s), especially polymer membrane, or one or more non-woven substrate(s) with electrically-conductive coating(s) or layer(s), especially metal coating(s) or layers(s) or respectively coating(s) or layer(s) formed by other conductive material, in particular on at least one site of the one or more filter membrane (s), especially polymer membrane(s). Especially, the metal coating or layer is porous as well. Alternatively, the porous surface may be provided by at least one metallic membrane. Preferably, gold or platinum is used as material for the electrically-conductive metal coating(s) and/or layer(s). Preferably, the metal used is pure and/or the coating or layer consists of solely a single metal. Especially, the polymer membrane itself is non-conductive and only the coating is electrically conductive. Especially only one kind of conductive material and/or metal is used for the charged porous surfaces. In particular, the electrically conductive coating or layer is not formed by a metallic net, in particular no metallic net coated with gold or platinum. This is particularly advantageous for pleating or winding - as described later in this disclosure - using at least one membrane as the porous surface(s), said at least one membrane being coated, especially coated with metal, most preferably a metal coating using gold or platinum as the metal.

The term "charged surface" means that the surface possesses a charge on its surface, in particular with a distinct polarity. In this context, a chemically charged surface is, e.g., achieved by ionic groups and/or compounds being placed at the surface, in particular, via chemical bonding of, e.g., quaternary ammonium groups, in particular for achieving a positively charged surface, or carboxy methyl groups, in particular for achieving a negatively charged surface, or of compounds containing such to the surface. For this purpose, an anion-exchange membrane or a cation-exchange membrane may be used as the charged porous surface, in particular with an additional electrically-conductive, in particular, e.g., metallic, coating or layer on the surface. Especially, an electrically charged porous surface, may be achieved by applying a, in particular base, voltage, which is applied to the electrically-conductive, especially metal, coating or layer or the membrane itself, if said membrane is electrically-conductive, e.g., if a metallic membrane is used. In particular, electrically charging the surface can be used to adjust the charge of a chemically charged membrane, thus to enhance or decrease the overall charge of the charged porous surface. Especially, the porous surface is not formed by a gel or resin. In Particular, the charged surface can also be a chemically and electrically charged. In this regard, a chemically charged surface, e.g., an ion exchange membrane, is provided in step a) with an electrically conductive coating disposed on the surface, especially membrane, to which the base voltage is applied. Thereby the overall charge of the charged surface may then be constituted by a combination of the chemical charges disposed and electric charges applied. Hence, the electric charge applied via the base voltage may be used to shape, influence or modify the chemical "pre-charge" of the surface. In this regard, the applied electric charges can be of the same polarity thereby enhancing the "pre-charge". If an opposite polarity is used, the "pre-charge" may be decreased or even nullified or the polarity of the overall charge of the charged surface may be switched to the opposite polarity compared to the "pre-charge".

In particular, for applying voltages, especially the release and/or base voltage, the electrically conductive charged porous surface and/or the electrically-conductive coating or layer of the charged porous surface is used as a first electrode and/or the first electrode may be part of the charged porous surface and additionally, a second electrode is provided as a counter electrode. The second electrode may be formed by a further electrically-conductive coating or layer as well. Especially, the second electrode may preferably be provided on a second side of the porous surface opposite to a first side of the porous surface on which the electrically conductive coating or layer forming the first electrode is located. In this regard, the first and second electrode may be disposed on different porous surfaces, in particular membranes. For example, the first electrode may be established by a conductive coating of a first porous membrane and the second electrode by a conductive coating of a second porous membrane.

In Particular, voltages applied, especially base and/or release voltages, are in the range from 0,1 to 50 volts, preferably from 0,1 to 3 volts, in particular to 2.5 and/or above 2 volts volts. In particular voltages exceeding 3 volts, in particular in some embodiments exceeding 2 volts, are solely applied for short amounts of time, in particular usually for less than 2 seconds.

In Particular, during, and especially as well prior to, step d, further fluid, which might be of the same or different kind, in particular with the given pH value and/or with pH value differing form the given pH value by less than 1, in particular 0.5, in particular 0.2, in particular 0.1, and/or not containing any species, is brought in contact with the charged porous surface. At least parts of said further fluid may be passed over and/or through the porous surface. Furthermore, said fluid, especially that passed over and/or through the porous surface, in step d) is collected and contains species, in particular (bio-)molecules or vectors containing (bio-)molecules, released within step d), in particular only one single specific species, in particular (bio-)molecules of same or identical type or vectors of a single group of vectors, thereby selective separating said one single specific species from the other remaining specific species, in particular said biomolecules of a single type from different biomolecules or said one group of vectors from the other vectors. A single specific species, in particular single group of vectors is in particular homogenous and/or contains specific molecules or a group of vectors with a specific isoelectric point, which is in particular a distinct isoelectric point and especially is not described with a range of isoelectric points and/or is not a range of isoelectric points, and thus especially consists of species which are identical, in particular identical molecules or vectors containing molecule of identical type with identical molecule contained and/or molecule contained in an identical amount. In this context, the term identical does not necessarily imply exactly identical but includes essentially identical as well, especially contains molecules/vectors which a person skilled in the art will consider identical, e.g., due to same molecular weight, same functionality and/or functional groups, e.g., for medical applications without the need of enantiomeric purity, different enantiomers of the same molecule.

The pH value differing form the given pH is preferably in the range of pH°6 to pH°9, preferably in the range of pH°6.5 to 8.5, even preferred in the range of pH°7 to pH°8, which is in general least harmful to species that might be therapeutically used or happen to naturally exist within the human body.

In particular, steps b) and c) may be performed repeatedly in form of multiple cycles, especially prior to performing step d). Performing such multiple cycles, may be used for enrichment of desired species being adsorbed to the membrane. Usually, within preparation of the species, concentrations in the fluid are low.

Consequently, the used fluid containing species is usually high in amount to bind a desired amount of species but may be reduced by using less further fluid in step d) and/or letting most of the fluid used pass over and/or through the surface in step c) while removing the fluid brought in contact, passed over and/or through with subsequent release in step d) into fluid with lesser volume.

Additionally, all steps b) to d) may be used in multiple cycles as well. This may be beneficial if several different impurities are present resulting from the preparation of the desired species. E.g., vectors containing molecule with an undesired amount of molecule(s) contained or respectively containing no molecule at all or containing an undesired molecule contained may be accounted as an impurity in this regard. Those are to be separated from a desired vector containing molecules. Generally, pure products are especially needed with respect for (bio-)molecules, in particular for those intended for medicinal applications.

By performing multi cycles, e.g., in a first cycle, a first impurity may be selectively separated from the desired vectors with a second impurity not being separated. Then, in a second cycle with adjusted process conditions, e.g., at another given pH value and/or with adjusted voltages, it may be possible to separate the desired vectors from the second impurity. Furthermore, if the selective separation is incomplete, by performing multiple cycles, separation with higher purity might be achieved.

Especially, with respect to vectors containing molecule, the at least two groups of vectors may differ in the amount of molecule contained. Consequently, the first group of vectors contain molecule in a first amount and that the vectors of the second group of vectors contain molecule in a second amount.

In an - especially in view of gene therapy - advantageous embodiment, the molecule contained in the vectors is a genome, in particular RNA or DNA. Advantageously, one group of the at least two groups of vectors may then consist of vectors containing a full genome, while at least one other group of the at least two groups of vectors consists of vectors containing a partial genome and/or no genome. Especially, vectors containing a partial genome and vectors containing no genome can form individual groups of vectors but may also be accounted as a single group of ranges for which the range of isoelectric points and net overall charges must be chosen accordingly.

In an alternative embodiment the molecule contained can be a drug or a protein or another biologically active substance.

With respect to pH values, acidic or alkalic pH values may act harmful on biomolecule(s) in general. E.g., proteins may show agglomeration or alike at acidic pH values, e.g., at pH values lower than pH 5, in particular using buffer solution having those pH values. Human blood, e.g., has roughly pH 7.4, thus a close to neutral pH value. Hence, neutral pH values are preferably used for all fluids used within the method of this disclosure, in particular between pH 6 and pH 8, most preferably pH°7.4. Advantageously, the pH value is and/or remains constant throughout all of steps b) to d), in particular for all fluids used within the method and/or at and/or surrounding the charged porous surface and/or fluids at and/or surrounding the charged porous surface. In this context, pH values with negligible differences are referred to as constant, e.g., pH values equal to the first decimal point. In this regard, any voltage applied may induce local pH changes, e.g., due to species being locally protonated or deprotonated. Such effects are negligible with respect to a global pH value of the fluids, which are therefore still referred to as constant. However, while performing cycles, especially full cycles comprising of steps b) to d), the pH value may be held constant for a single cycle but be adjusted between cycles, e.g., by performing a first cycle at pH°8 and a second cycle at pH°6. In this regard, a change in pH value is not used to perform any release of previously adsorbed species. However, pH values may be chosen with close proximity to the isoelectric point of any of the amphoteric species, especially vectors, adsorbed but such that no release is caused, particularly may be adjusted accordingly between cycles, in particular with respect to different species, especially vectors, to be released within any of the cycles. In this regard, choosing pH values in close proximity (especially with a difference between the pH° value and the isoelectric point of less then 1, especially less than 0,5) to the isoelectric point of any specific of the species to be released, especially vectors, but such that no release is caused, may in general particularly lower the release voltage needed for initiating release of said specific species, especially vector, in step d).

In particular, no, in particular no highly concentrated, salt solutions are used to perform any release of previously adsorbed species, especially if the specie(s) is/are biomolecules, e.g., vectors. In particular, all fluids used are fluids "free of salts". In this context, liquids with salt ion concentrations being negligible are considered "free of salts". In particular, common pH buffer solutions for pH 6 to pH 8 are considered "free of salts" within the context of the disclosure. In this regard, liquids, in particular all liquids, introduced within the method preferably may be or comprise such buffer solutions used for maintaining constant pH values and/or ions in small amounts and/or salts in small amounts, preferably below 150 mM. As such, those buffer solutions contain salts in small amounts with those amounts being low compared to concentrations of salt solutions commonly used for common salt elution. Especially, those buffer solutions, e.g., phosphate-buffered saline (PBS), may be in particular isotonic and thus provide osmolarity and ion concentrations which match those of the human body, which may be particularly beneficial for medical related applications, especially with respect to biomolecules, e.g., vectors, as specie(s). In particular PBS used in common concentrations usually provides isotonic conditions, in particular with osmolarity and ion concentrations which match those of the human body. In particular, the release of species in step d) is solely performed by applying the release voltage. However, changing the pH value may be used for cleaning purposes, e.g., if any undesired impurities remain bound to the surface after separation and/or recovery of the desired species, in particular vectors, especially subsequent to step d), in particular, if the method is performed in cycles, after step d) of the last cycle.

According to the invention, the, in particular selective, separation of the at least two species is performed by
I. in step b), selectively adsorbing at least parts of the first species of the at least two species, in particular of the first group of vectors of the at least two groups, while at least one other species of the at least two species, in particular at least one other group of vectors of the at least two groups, in particular no other species, in particular no other group of the at least two groups, is absorbed); and/or
II.
   i.) in step b), adsorbing the first and at least parts of at least a second species of the at least two species, in particular the first and at least a second group of vectors of the at least two vectors, in particular all species of the at least two species, in particular groups of the at least two groups of vectors, and
   ii.) in step d), selective releasing the first species, in particular the first group of vectors, wherein the at least one other species, in particular group of vectors, adsorbed in step b) remains, in particular all other species, in particular all other groups of vectors, adsorbed in step b) remain, adsorbed.

In this regard, selectivity in the separation based on option I. is not limited to solely adsorbing species being first species but more than one species may be absorbed in step b). In this regard, at least one species of the at least two species must not be absorbed in step b) to achieve selectivity based on selectivity concept I. Hence, option I. particularly includes absorbing a group of species consisting of more than one species, in particular adsorbing a first group of species, while at least one other species, in particular a second group consisting of one or more further species different from those of the first group of species, is not adsorbed. In this regard, the first and second group of species are selectively separated. However, for selectively separating the species contained within the first group, such method may then additionally use a selective desorption according to concept II. within step d) and/or may be performed in multi-cycles, for which, e.g., general process conditions may be adjusted within between cycles, e.g., cycles using different given pH values or alike.

In Particular, with respect to separation of groups of species, in particular groups of vectors, of the at least two groups of species, in particular vectors, the first group of species, in particular vectors possess a first isoelectric point and/or range of first isoelectric points and/or a first overall charge and/or range of first net overall charges at the given pH value and a second group of species, in particular vectors, possess a second isoelectric point and/or range of second isoelectric points and/or second net overall charge and/or range of second net overall charges at the given pH value.

Within an especially advantageous embodiment, the given pH is chosen so that, at the chosen given pH, a polarity of the net overall charge of one of the first and second species or first and second group of species, in particular vectors, of the at least two species or groups of species, in particular vectors, is opposite to the polarity of the charge of the charged porous surface and the net overall charge of the other of the first and second group is either zero or has the same polarity than the charge of the charged porous surface, thereby only adsorbing one of the first and second species or group of species, in particular vectors, in particular within steps b) and c). Within this embodiment, in particular, the selective separation occurs according to option I. Advantageously, solely the desired species, in particular the vectors containing the desired molecule or desired amount of molecule(s), is adsorbed or alternatively all but the desired species, in particular vectors containing the desired molecule or desired amount of molecule(s), are adsorbed, thus the desired species, in particular vectors containing the desired molecule or desired amount of molecule(s), being the only vectors not adsorbed to the surface in step b).

Advantageously, the given pH value is chosen so that, the given pH value is between the isoelectric point of the first species, in particular group of vectors and the isoelectric point of the second species, in particular group of vectors, respectively between the ranges of first isoelectric points and second isoelectric points, respectively between the first isoelectric point and the range of second isoelectric points and/or the second isoelectric point and the range of first isoelectric points. Especially, at such chosen given pH, the first and second species, in particular group of species, in particular vectors, differ with respect to the polarity of their net overall charge. Consequently, solely one specific species, respectively one group of species, in particular solely the vectors of a single group of the first and second group, are adsorbed in step b) due to one specific species, respectively group of species, in particular group consisting of vectors, having a polarity opposite to the charge of the surface and the other specific species, respectively group of species, in particular group consisting of vectors, having the same polarity as the charge of the surface. Since only species with a polarity opposite to the charge of the surface can be adsorbed, one of the species, in particular groups of species, in particular groups of vectors, is selectively adsorbed to the surface while the other of the first and second species, respectively group is not adsorbed to the surface and is in particular removed in step c) together with the fluid brought in contact with, passed over and/or through the surface.

Within another especially advantageous embodiment, both the first and second species, respectively group of species, in particular vectors, in particular all species, in particular groups of species, in particular vectors, are adsorbed to the charged porous surface. This may correspond to a selective separation according to option II. Advantageously, the given pH value is chosen so that, the chosen pH value is either above or below both isoelectric points, respectively ranges of isoelectric points, of the first and second species, respectively group of species, in particular of all species, respectively groups of species, in particular vectors. Especially, the net overall charges both the first and second species, respectively groups, in particular for vectors of both the first and second group, in particular all species, respectively groups, possess the same polarity at the given pH value, said polarity being opposite to the polarity of the charge of the charged porous surface.

Advantageously, the release voltages for adsorbed first and second species, respectively groups of species, in particular adsorbed vectors of the first and second group, differ for releasing those within step d). By applying a first release voltage to the electrically conductive surface and/or the electrically conductive coating or layer, the net interaction force for one of the first and second species, respectively group, in particular of vectors, in particular a single group of all groups of species, in particular vectors, is switched to repulsive while the net interaction force for the other of the first and second species, respectively group, in particular all groups, still adsorbed to the surface, remains attractive, thereby selectively desorbing solely the first or second species, respectively group, in particular of vectors, with the other species, respectively group, in particular all other groups, remaining adsorbed to the charged porous surface.

Subsequently, after desorbing one of the first and second species, respectively group of species, in particular vectors, respectively group of vectors, previously adsorbed by applying the first release voltage, advantageously, the voltage applied is adjusted to a second release voltage, wherein by applying the second release voltage, the net interaction force of the other of the first and second species, respectively group, in particular of vectors, is switched from attractive to repulsive, thereby desorbing the other of the first and second species, respectively group, in particular of vectors. In particular, if more than two species, respectively groups of species, in particular vectors, were adsorbed to surface, the first and second release voltage are chosen, so that a third species, respectively group of species, in particular vectors, may remain adsorbed while desorbing the first and second groups of vectors.

In another advantageous embodiment, the porous surface is electrically charged by applying a base voltage. Desorption in step d) may then be initiated by changing, particularly decreasing, the base voltage to the release voltage, in particular by stepwise changing, e.g., decreasing, the voltage to the first release voltage and subsequently to the second release voltage. "Changing or Decreasing of voltage" may include a switch of polarity, hence, e.g., a, in particular step-wise, degree from a negative voltage to a positive voltage or the other way around.

Especially, it may be beneficial to combine options I. and II. for achieving a selective separation, so that - with more than two species, respectively groups of species, in particular vectors present - dedicated species, respectively groups of species, in particular vectors, are selectively adsorbed in step b), with respectively the remaining species, respectively groups, selectively being not adsorbed in step b), and later, of the dedicated species previously adsorbed in step b), at least one species is selectively released in step d), in particular all species are stepwise released in step d).

In yet another beneficial embodiment, at least two charged porous surfaces are provided in step a), in particular formed by polymer membranes. Those at least two charged porous surfaces comprise at least a first and a second charged porous surface, in particular polymer membrane. Moreover, the first charged porous surface is the electrically conductive and/or comprises an electrically conductive coating or layer. Optionally, the second charged porous surface is electrically conductive and/or comprises an electrically conductive coating or layer as well. E.g., by having more than one charged porous surface, the overall porous surface may be enhanced, hence in particular yielding higher capacities for binding species to the surface(s), especially if combined with a stacked and in particular pleated arrangement, especially according to the disclosure to be found further below.

Preferably, the at least two charged porous surfaces - especially if those are formed by polymer membranes - are arranged as stacked layers building a stack. In such arrangement, the stack is formed such that, the first charged porous surface and/or the electrically conductive coating or layer of the first charged porous surface is placed on a first end of the stack. The counter-electrode is preferably disposed on a second end opposite to the first end of the stacked layer. Moreover, the, especially a multitude of, second charged porous surface(s) is preferably placed between the first porous surface and the counter-electrode. Particularly advantageous, the second charged porous surface is preferably not electrically conductive nor comprises an electrically conductive coating or layer and is in particular chemically charged. Alternatively, the second charged porous layer may be electrically conductive and/or comprises an electrically conductive coating or layer. Then, the counter-electrode is preferably formed by the second charged porous surface itself or the electrically conductive coating or layer of the second charged porous surface. Hence, in such arrangement, the second charged porous surface is placed at the second end of the stack.

Advantageously, no further electrodes are placed in between the first and second charged porous surface, in particular all charged porous surfaces and/or of the surfaces(s) forming the stacked layer, solely surfaces placed at the first or second end are electrically conductive and/or comprise an electrically conductive layer. In Particular, at least one of the polymer membranes may act as an insulator between the electrically conductive coatings or layers. Alternatively, respectively additionally, a distinct insulator, which, e.g., may be formed by a further polymer membrane, is placed between the electrically conductive coatings or layers. Thus, the insulator may in particular be formed by at least one, especially a multitude of, in particular up to 30, preferably between 5 and 15, additional polymer membrane(s) and/or a non-conductive side(s) of polymer membrane(s) comprising electrically conductive coating(s), in particular the non-conductive side(s) of the membranes forming the first and second surface.

In particular a stacked arrangement of charged porous surfaces, in particular polymer membranes may provide high surface areas with respect to space needed for providing the surfaces, in particular membranes, within any suitable apparatus being used for performing the method according to the disclosure. In particular such arrangement may also provide fluid permeable channels between the surfaces, in particular membranes, e.g., by the surfaces, in particular membranes being slightly spaced form another. Such arrangement, may particularly enable the fluids to be passed over and through the surfaces simultaneously.

In particular, stacked arrangements as disclosed may preferably consist of a multitude of surfaces, in particular membranes, in particular consist of at least 2 membranes, in particular up to 50 membranes, preferably between 5 and 20 membranes.

In yet another advantageous embodiment, the first porous surface, in particular polymer membrane, possesses or is charged with a first polarity. Moreover, the second porous surface possesses or is charged with a second polarity. Preferably, the second polarity is in particular opposite to the first polarity. The polarity of the first and second charge, is the polarity of the respective overall charge, which may be provided and/or defined by the sum of a chemical charge as well as an electrical charge applied to the porous surface by means of the base voltage. Especially, usage of more than one charged porous surfaces may provide several beneficial effects, especially charged porous surfaces with different properties, especially different charges, in particular charges with different polarity.

A particularly beneficial embodiment is based on using two charged surfaces of which both the first and second charged porous surface are preferably both formed by chemically charged polymer membranes, in particular ion-exchange membranes, e.g., a cation- and an anion exchange membrane in particular. Preferably, both polymer membranes, in particular ion-exchange membranes, possess electrically conducting coatings or layers on at least one site of each of the polymer membranes, in particular ion-exchange membranes. Moreover, each membrane may possess a further electrically conducting coating or layer on at least a second site of the membrane, in particular the second site being located opposite to the first site. By using two charged surfaces with different polarity, species being anionic at the given pH can be absorbed to one of said surfaces, in particular the anion exchange membrane, while species being cationic can be absorbed to the other surface, in particular the cation exchange membrane. This may especially be beneficial, if the given pH is chosen so that the given pH is between the isoelectric points of at least two species to be separated, in particular if it is desirable to separate both of those species, in particular for performing subsequent methods, e.g., concentration enhancement, detection enhancement or if both of those species are to be understood as impurities, e.g., in purification of blood or alike, in particular purification of blood plasma or separation of (blood) plasma protein(s).

Within such method using surfaces with different polarities, especially anion and cation exchange membranes, while performing step b) of the method in disclosure, species with a specific net over all charges with having the second polarity are adsorbed to the first porous surface and species with a specific net overall charges with having the first polarity are adsorbed to the second porous surface, e.g. species with cationic overall charge at the given pH to the cation exchange membrane and species with anionic overall charge at the given pH to the anion exchange membrane. In subsequent step d), release voltage is preferably applied to the first charged porous surface while no release voltage is applied to the second charged porous surface. Thereby, only species previously adsorbed to the first charged porous surface are desorbed desorbing, with while species adsorbed to the second porous surface remain adsorbed. Optionally, in particular after collecting the fluid to which the species were eluted to is collected and/or removed, it may be beneficial to recover the other species still absorbed to the second surface by applying release voltage to the second charged porous surface afterwards, thereby desorbing species previously adsorbed to the second charged porous surface. Again, the fluid to which such species were eluted to may also be collected and/or removed.

In particular, if more than two species are to be selectively separated it may be beneficial to combine several of the previously described embodiments. E.g., a method using both an anion exchange membrane and a cation exchange membrane may combine both options I. and II. for achieving selective separation. In this regard, the given pH value may be chosen so that, two species are adsorbed to the same porous surface with a third species being adsorbed to another porous surface. Consequently, if the surfaces are eluted separately, the third species is efficiently separated from the other two species. Such approach uses option I. for selectivity. For separation of the remaining two species bound to the same porous surface, option II for achieving selective separation may be used. Hence, the release voltage may be adjusted step-wise to ensure stepwise elution of the two species bound by selective desorbing one of those species with a first release voltage which is not sufficient to initiate desorption of the other species bound to the surface, thereby selective separating said species from another. Alternatively, for separating those two species bound to the same surface, yet another cycle of the method may be performed. In this regard, the given pH may be amended between cycles, in particular to amend the net overall charges of the species. Preferably, the pH value is then chosen such that the net overall charges of those species then possess a different polarity. By doing so, in the second cycle, those species may then be adsorbed to different surfaces within step b) of the second cycle. Consequently, again option I. for selective separation was used.

Additionally, within such approach using differently charged porous surfaces, it may be particularly advantageous to provide conductive coatings or layers to be used as electrodes for applying the release voltages between each of the porous surfaces, in particular membranes. Thereby, applying the release voltages to solely a part of the surfaces may be enabled. Especially, the porous surfaces, in particular the polymer membranes, may then be integrally used as the insulator between the conductive coatings or layers.

Especially, every part of the fluid(s) is passed over every of the at least two charged porous surfaces, especially if those are different charged porous surfaces, in particular first charged porous surface(s) with the first polarity and second charged porous surface(s) with the second polarity opposite to the first polarity. When a multitude of identical charged porous surfaces is used one might pass each part of the fluid(s) over every charged porous surfaces or only some charged porous surface(s).

Preferably, for each elution the fluid is collected and removed before performing the next elution in general.

In yet another aspect, advantageous arrangements of surface(s), in particular membrane(s), are provided. Such advantageous arrangements are a pleated arrangement of surface(s), in particular membrane(s), and/or a wound arrangement of those. Such arrangements are particularly useful for chromatographic methods, in particular including but not limited to the method according to the above disclosure. In particular such arrangement may be used as part of any suitable chromatographic apparatus.

In this regard, the pleated arrangement is generated by folding a flat surface or alternatively a stack of surface(s) into a multitude of pleats. With respect to the wound arrangement, the flat surface or stack of surface(s) is wound into a series of wound stacked layers. The pleated or wound arrangement is placed within a housing. Generally, the housing may be chosen to host the pleated or wound arrangement such that fluids may be passed over and/or through the surface(s), preferably membranes. The housing may provide for a distinct direction of flow, in particular provided by at least one inlet and at least one outlet for feeding (inlet) and removing (outlet) fluids. The pleated or wound arrangement may then be disposed in the housing such that, it blocks free passage from inlet to outlet and/ or ensures any flow from the inlet to the outlet will pass through and/or over the membrane at least once, preferably multiple times and/or at and/or through multiple, preferably disjunct, sections or areas of the stack and/or membranes. The pleated and/or wound arrangement may be disposed in the housing such that, a main extension of the arrangement is parallel, perpendicular or oblique to said distinct direction of flow.

Tube-like structures may act as housing for the arrangements. Such tube-like structures may possess base areas in any form, including but not limited to rectangular or circular base areas. Moreover, suitable housing may be in the form box-like structured housings providing inlet(s) and outlet(s) between which the charged porous surface(s) is/are placed. The charged porous surface(s) between those inlet(s) and outlet(s) may be fed laterally. In this regard, the charged porous surface(s) is/are disposed such that, free passage from inlet to outlet is blocked and such that, fluid being fed through the outlets must pass through and/or, preferably and, over the charged porous surface(s), preferably membrane(s) at least once. Consequently, the inlet(s) must be oriented on a first side of the charged porous surface(s) with the outlet(s) being arranged on a second site of the charged porous surface(s), opposite, preferably diagonally opposite, to the first side.

However, circular housings consisting of an inner core and an outer cage with gap in between the core and the cage are preferred in other use scenarios. In this regard, the gap has a gap distance defined by the shortest distance between the core and the cage. The pleated or wound surface, in particular membrane, is placed in the gap. Preferably, the inner core and outer cage are permeable to fluids and/or comprise the inlets/outlets for feeding fluid to the surface(s) and/or removing fluids/eluate. Preferably the fluid needs to pass radially through the arrangement in the gap to flow from inlet to outlet. Generally, only one of the inner core and outer cage has inlets with the other having outlets. In this regard, an inlet may describe any, in particular fluid permeable, passage for feeding, respectively an outlet any passage for removing. In particular, volume inside the core may be used for either feeding or removing.

With or without pleating and/or winding, in case surfaces with different polarities are used preferably the fluid is passed over all kind of surfaces with different polarities, particularly sequentially, and/or they might be arranged in a way that fluid passing from the inlet to the outlet is passed over all kind of surfaces with different polarities, particularly sequentially.

Preferably, polymer membrane(s) are used for providing and/or as the surface(s) in pleated and/or wound arrangements. Most preferably, the pleated and/or wound arrangement is/are formed by one than one polymer membrane. Of those polymer membranes, at least some or all are preferably coated with a metal coating or layer on at least one side of the membrane(s). Generally, the pleated and/or wound arrangement, preferably stack of membranes comprises at least two conductive coating or layers, preferably metal coatings.

In a particular advantageous embodiment, the pleated and/or wound arrangement consists of at least two polymer membranes with a metal coating as the conductive coating or layer on one side of the respective membrane(s) forming a stack, respectively with further, in particular non-conductive polymer membranes, between the coated membranes. Preferably the metal coatings are arranged such that at least two are placed at the outermost sides of the stack, especially the outer sides of the outermost polymer membranes within the stack, one on each side, especially with respect to the flow the flow of direction within the housing. In this regard, it is especially beneficial to pleat and/or wind the membrane(s) and the conductive coating(s) together. In particular, polymer membranes with metal coatings, in particular gold or platinum, may be pleated or wound, in particular together. Preferably the wound and/or pleated stack consists of only one type of membrane, being uncoated, coated and/or having binding sited attached, e.g., differing only by coating/non-coating and/or binding sites.

In yet another advantageous embodiment, each polymer membrane of the stack may have metal coating as the conductive coating or layer on one or both side(s) of the respective membrane(s). Preferably, the or some membranes themselves are non-conductive and act as an insulator between the metal coatings or layers. In particular, release voltage may only be applied to selected metal coating(s) and/or layer(s) one at a time, with in particular no release voltage being applied to at least one other metal coating and/or to different pairs of metal coatings, each pair of pairs might share one metal coating.

Preferably, the stack is formed of "equal membrane(s)", hence membranes of the same type. Of those, at least some preferably comprise conductive coating(s) or all comprise conductive coating(s). Preferably, some of those may comprise conductive coating(s) while others preferably may not.

However, in yet another advantageous embodiment, "different membranes", in particular with different polarity of charge of the charged surfaces, may be used in combination, e.g., cation and anion exchange membranes. In this regard, providing arrangements in which voltages, especially release voltage, may be applied to one type of membrane disjunct from other type(s), may be beneficial. In this regard, of membranes of any type, at least one of those membranes of said type particularly has a conductive coating or layer. Between the electrically conductive coatings or layers of the types at least one insulator, especially formed by any of the polymer membranes, is provided. Said arrangement may be used to selectively apply release voltages to selected membrane(s) or membrane(s) of selected type.

Preferably, with respect to all embodiments of the pleated and/or wound arrangement formed by stack, the stack is pleated and/or wound as a whole, in particular all membranes and electrically conductive coatings are pleated and/or wound together.

Using a pleating or winding assistant and/or promoter may be beneficial as well, especially for achieving an easier preparation of the pleats/windings as well as maintaining a shape of the pleats/windings prepared by pleating or the stacked layers formed by winding after such pleating/winding. Such assistant(s) and/or promoter(s) may consist of one or more thermoplastic layer(s), which is heated during preparation of the pleats/windings, in particular to temperatures between 30°C and 100°C and/or above the glass transition temperature, and will retain the pleats and/or winds, especially their shapes, after said preparation, especially at temperatures below aforementioned temperatures. Preferably, the pleating and/or winding assistant and/or promoter is porous and/or permeable to fluids as well.

By pleating or winding the surface(s), in particular membranes(s), higher surface areas per volume may be provided compared to unpleated or non-wound, hence flat surface(s), in particular membrane(s), especially when taking into account the housing.

The surface area to volume ratio may be influenced by choosing a form of the multitude pleats, especially with respect to the housing. In this regard, the pleats consist of flat areas located between local folding points, with each flat area having a length, the pleats preferably having a M-shape as the from. For each pleat, preferably a first length is chosen to surpass at least one extension, especially width, diameter or alike, of the housing, especially, for circular housing with outer cage and inner core the distance of the gap. In this regard, due to the core having a smaller circumference compared to the cage, pleats with regular, thus at least near identical lengths of surface areas will lead to lower, at least non-optimized, surface area to volume ratios. Hence, pleats with irregular M-shape are preferred, since those forms yield higher, especially optimized surface area to volume ratios. Consequently, such irregular M-shapes comprises flat areas with at least one second length differing from the first length. Most preferably, pleat sets consisting of more than one pleat are used. Those pleats of any pleat set preferably comprises pleats with at least two different irregular M-shapes. In this regard, the at least one second length may smaller than the distance of the gap or width of the channel. Most preferably, the pleat sets are, in particular regularly repeated, within the housing. In particular, for circular housing, the pleats and/or pleat sets form a, in particular full, circle within the housing.

With respect to wound arrangements, circular housings are beneficial as well. The wounds may be formed by wrapping the surface(s) or stack of surface(s) around the inner core of the housing, thereby the inner core in particular supports the winds formed by wrapping.

## Claims

1. Method for selective separation of amphoteric species, in particular biomolecules, wherein the species consists of at least two specific species, each specific species possessing a specific isoelectric point, thereby having a specific net overall charge at a given pH value;
the method comprising the following steps:
a) providing at least two chemically charged porous surfaces, an electrode and a counter-electrode, wherein the at least two charged porous surfaces, the electrode and the counter-electrode are arranged as stacked layers building a stack, wherein the electrode is formed by a first electrically conductive coating or layer placed on a first end of the stack, wherein the first electrically conductive coating or layer is either integrally formed to and/or within a first charged porous surface of the at least two charged porous surfaces or formed as a distinct, particularly non-integral layer, disposed on said first charged porous surface;
b) bringing a fluid with the given pH value in contact with the charged porous surface, the fluid containing said amphoteric species, thereby adsorbing at least a first species of the at least two species to at least one of the at least two charged porous surfaces and/or to at least the first charged porous surface due to an interaction force between the net overall charge of the adsorbed species and the at least one of the at least two charged porous surfaces and/or the first charged porous surface, the interaction force being attractive;
c) preferably passing at least parts of the fluid over and/or through the at least two charged porous surfaces;
d) applying a release voltage to the electrode, the release voltage being chosen, so that the net interaction force between the at least one of the at least two charged surfaces and/or the first charged porous surface and at least parts of the adsorbed species is switched from attractive to repulsive, thereby releasing said parts of the adsorbed species;
**characterized in that** the separation is performed by
I. in step b), selectively adsorbing at least parts of the first species while at least one other species of the at least two species is not adsorbed, in particular no other species of the at least two species is absorbed; and/or
II.
i.) in step b), adsorbing at least parts of the first and at least parts of at least a second species of the at least two species, in particular all species of the at least two species, and
ii.) in step d), selectively releasing the first species, wherein the at least one second species adsorbed in step b) remains adsorbed, in particular all other species adsorbed in step b) remain adsorbed.

2. Method according to claim 1, wherein the at least two charged porous surfaces are formed by ion-exchange-membranes, in particular anion- or cation-exchange membranes.

3. Method according to any of the preceding claims, **characterized in that** the counter-electrode is disposed on a second end opposite to the first end of the stack,
wherein a, especially a multitude of, in particular up to 30, in particular preferably between 5 and 15, second charged porous surface(s) of the at least two charged porous surfaces is placed between the first porous surface and the counter-electrode,
wherein particularly no further electrodes are placed in between the first and second charged porous surface(s) and/or in between the second charged porous surface(s), in particular in between all charged porous surfaces.

4. Method according to any of the preceding claims, wherein the counter-electrode is formed by a second electrically conductive coating or layer, wherein the second electrically conductive coating or layer is either integrally formed to and/or within a, in particular the, second charged porous surface of the at least two charged porous surfaces and/or one of said second charged porous surfaces, particularly placed at the second end of the stack, or formed as a distinct, particularly non-integral layer, disposed on said second charged porous surface and/or said one of said second charged porous surfaces, particularly placed at the second end of the stack.

5. Method according to any of the preceding claims, wherein of the at least two charged porous surfaces(s) forming the stacked layer, solely charged porous surfaces placed at the first or second end, in particular the first and second charged porous surface, comprise an electrically conductive coating or layer and/or
wherein an insulator is placed between the first and/or second electrically conductive coating or layer and/or the counter-electrode and respectively the first and/or second charged porous surface,
in particular wherein the insulator is formed by non-conductive side(s) of polymer membrane(s) comprising the first and/or second electrically conductive coating(s) or layer(s).

6. Method according to any of the preceding claims, wherein during step d, further fluid, in particular with the given pH value and/or with pH value differing form the given pH value by less than 1, in particular less than 0.5, in particular less than 0.2, in particular less than 0.1, and/or not containing any species, is brought in contact with the at least two charged porous surfaces,
wherein at least parts of the further fluid is passed over and/or through the at least two porous surfaces and/or the stack,
wherein the fluid passed over and/or through the at least two porous surfaces and/or the stack in step d) is collected and contains species released within step d).

7. Method according to any of the preceding claims, **characterized in that** the pH value is held and/or remains constant and/or differs by less than 1, in particular less than 0.5, in particular less than 0.2, in particular less than 0.1, throughout all of steps b) to d), in particular for all fluids.

8. Method according to any of the preceding claims, **characterized in that** the given pH is chosen so that, the given pH value is between the isoelectric points of the first and second species and that at the chosen given pH, a polarity of the net overall charge of the first species is opposite to the polarity of the charge of the at least two charged porous surfaces and/or the first charged porous surface and that the net overall charge of the second species, in particular all other species, is either zero or has the same polarity than the charge of the at least two charged porous surfaces and/or of the first charged porous surface, thereby only adsorbing the first species to the at least two charged porous surfaces and/or to the first charged porous surface and in particular no other species in step b).

9. Method according to any of the preceding claims, **characterized in that** the given pH value is chosen so that, the chosen pH value is either above or below both isoelectric points of the first and second species and so that polarities of the net overall charges for both the first and second species are of equal polarity, said polarity being opposite to the polarity of the charge of the at least two charged porous surface and/or the first charged porous surface, thereby adsorbing both the first and second species to the at least two charged porous surfaces and/or to the first charged porous surface in step b),
wherein the release voltage differs and/or is chosen differently for adsorbed first and second species in step d), wherein the net interaction force for one of the first and second species is switched to repulsive by applying a first release voltage to the electrode, while the net interaction force for the other of the first and second species remains attractive, thereby selectively desorbing solely the first or second species with the other remaining adsorbed to the at least two charged porous surfaces and/or to the first charged porous surface,
particularly wherein after desorbing one of the first and second species by applying the first release voltage, the voltage applied is adjusted to a second release voltage, wherein by applying the second release voltage, the net interaction force of the other of the first and second species is switched from attractive to repulsive, thereby desorbing the other of the first and second species.

10. Method according to any of the preceding claims, **characterized in that** the electrode and/or the counter-electrode and/or the first and/or second electrically conductive coating(s) or layer(s) are formed by and/or is/are a metal coating(s) or layer(s), preferably gold or platinum, in particular consisting of a single metal, preferably gold or platinum and/or **in that** the electrically conductive coating(s) is/are formed on a non-conductive carrier and/or non-conductive carriers.

11. Method according to any of the preceding claims, **characterized in that** the first porous surface possesses a first polarity and a second charged porous surface of the at least two charged porous surfaces, in particular the second porous surface, possess(es) a second polarity, the second polarity being opposite to the first polarity, in particular the first charged porous surface being formed by either of an anion- or cation-exchange-membrane and said second charged porous surface being formed by the other of anion- or cation-exchange-membrane, particularly wherein in step b), species with specific net overall charges having the second polarity are adsorbed to said first porous surface and species with specific net overall charges having the first polarity are adsorbed to said second porous surface.

12. Method according to any of the preceding claims, wherein the stack consists of and/or the at least two charged porous surfaces are formed by a multitude of surfaces, in particular membranes, in particular consist of at least 2 membranes, in particular up to 50 membranes, preferably between 5 and 20 membranes.

13. Method according to any of the preceding claims, **characterized in that** the stack is pleated.

14. Method according to any of the preceding claims, wherein the release voltage and/or the first and/or second release voltage is/are in the range from 0.1 to 50 volts, in particular 0.1 to 3 volts.

15. Method according to any of the preceding claims, wherein the amphoteric species are biomolecules or biomolecule-complexes, in particular amino acids, proteins, nucleic acids, in particular RNA or DNA, and/or wherein the method is used for separation of groups of species, in particular biomolecules, said groups of species particularly consisting of species of equal type, particularly with respect to biomolecules of equal medical applicability, in particular enantiomers having no enantiomer specific applications and/or variants of proteins being part of one protein family, and/or respectively possessing specific isoelectric points falling within a respective specific range of isoelectric points.
